# EUROPÄISCHE PATENTSCHRIFT

(11) **EP 3 060 268 B1**
(45) Veröffentlichungstag und Bekanntmachung des Hinweises auf die Patenterteilung: **21.11.2018**
(21) Anmeldenummer: 14789257.4
(22) Anmeldetag: 23.10.2014
(51) Int. Cl.: A61L 27/02, A61L 24/02

(54) **FORMSTABILE KNOCHENERSATZFORMKÖRPER MIT VERBLEIBENDER HYDRAULISCHER AKTIVITÄT**
SHAPE-STABLE BONE REPLACEMENT MOULDED ELEMENT WITH RESIDUAL HYDRAULIC ACTIVITY
ÉLÉMENT MOULÉ DE SUBSTITUTION OSSEUSE DE FORME STABLE AYANT UNE ACTIVITÉ HYDRAULIQUE RÉSIDUELLE

(30) Priorität: 23.10.2013 DE 102013221575
(43) Veröffentlichungstag der Anmeldung: 31.08.2016
(73) Patentinhaber: InnoTERE GmbH, 01445 Radebeul (DE)
(72) Erfinder: NIES, Berthold, 64407 Fränkisch-Crumbach (DE)
(74) Vertreter: Kailuweit & Uhlemann Patentanwälte Partnerschaft mbB
(86) Internationale Anmeldenummer: PCT/EP2014/072784
(87) Internationale Veröffentlichungsnummer: WO 2015/059240

(56) Entgegenhaltungen:
- WO-A2-2008/148878
- M. P. HOFMANN ET AL: "Carvable calcium phosphate bone substitute material", JOURNAL OF BIOMEDICAL MATERIALS RESEARCH PART B: APPLIED BIOMATERIALS, Bd. 83B, Nr. 1, 1. Januar 2007 (2007-01-01), Seiten 1-8, XP055169146, ISSN: 1552-4973, DOI: 10.1002/jbm.b.30761
- ANJA LODE ET AL: "Fabrication of porous scaffolds by three-dimensional plotting of a pasty calcium phosphate bone cement under mild conditions", JOURNAL OF TISSUE ENGINEERING AND REGENERATIVE MEDICINE, Bd. 8, Nr. 9, 30. August 2012 (2012-08-30) , Seiten 682-693, XP055169132, ISSN: 1932-6254, DOI: 10.1002/term.1563 in der Anmeldung erwähnt

## Beschreibung

Die Erfindung betrifft formstabile Knochenersatzformkörper aus mineralischem Knochenzement mit verbleibender hydraulischer Aktivität, ein Verfahren und ein Set zu dessen Herstellung sowie deren Verwendung als technische, medizintechnische und/oder pharmazeutische Produkte, insbesondere als alloplastisches Knochenimplantat.

Häufig ist bei der Verwendung von medizinischen Implantaten nur ein zeitweiliger Verbleib des Implantatmaterials im Körper erforderlich. Um bei derartigen Implantationen auf eine aufwendige Operation zur Entfernung eingesetzter Implantatmaterialien, die vom Körper nicht abgebaut werden können, zukünftig zu verzichten, wird seit vielen Jahren an der Entwicklung bioresorbierbarer Implantatmaterialien geforscht. Bioresorbierbare Materialien besitzen die Eigenschaft, nach der Implantation im Körper allmählich abgebaut zu werden. Die dabei entstehenden Abbauprodukte werden vom Körper weitgehend resorbiert.

Der Abbau von Implantatmaterialien kann aktiv oder passiv erfolgen. Beim aktiven Abbau durch den Organismus wird das Implantatmaterial durch enzymatische oder zelluläre Mechanismen degradiert. Dies trifft beispielsweise auf Implantatmaterialien aus Kollagen oder mineralischen Knochenzementen auf der Basis von Calciumphosphaten zu. Der aktive Abbau von Implantatmaterialien ist besonders dann erwünscht, wenn der Abbau im Rahmen des natürlichen Stoffwechsels erfolgt und nicht auf einer Entzündungsreaktion beruht.

Bisherige Lösungen für die Bereitstellung von alloplastischen Knochenersatzformkörpern basieren meist auf Calciumphosphaten, die durch Fällung oder über Hochtemperaturprozesse (Sinterung geeigneter Ausgangsmaterialien bei Temperaturen >500°C) hergestellt werden. Eine Herstellung über Hochtemperaturprozesse liefert zwar Materialien mit stofflicher Ähnlichkeit zu Knochenmineralien, führt aber zu starken Vergröberungen der Struktur, wodurch die Bioaktivität und Resorbierbarkeit eines Knochenersatzformkörpers beeinträchtigt werden.

Eine Sonderform stellen mineralische Knochenzemente dar, die über eine hydraulische Abbindereaktion *in situ* aushärten.

US 6,642,285 B1 offenbart einen hydraulischen Zement zur Herstellung von Knochenimplantaten, der aus drei aufeinander abgestimmten Komponenten besteht, die nach dem Mischen möglichst direkt zu einem makroporösen Feststoff aushärten sollen. Die erste Komponente stellt eine Calziumquelle dar, die in Verbindung mit einer wässrigen Lösung und einer hydrophoben Flüssigkeit bevorzugt innerhalb von 60 Minuten vollständig aushärtet, wobei das Produkt nach der Aushärtung keine hydraulische Aktivität mehr besitzt.

Hofmann et al. (J. Biomed, Mater. Res., 2007) offenbart Knochenersatzformkörper, welche dadurch erzielt werden, dass zunächst ein konventioneller Calciumphostzement mit wässriger Lösung gemischt und damit zur initialen Aushärtung gebracht wird. Danach erfolgt die Unterbrechung der Aushärtung durch Entfernen des Wassers. Anschließend erfolgt die Formgebung (Carving) und schließlich die endgültige Aushärtung (z.B. in situ).

WO 2008 148 878 A3 offenbart Pasten, Suspensionen oder Dispersionen bestehend aus einer resorbierbaren mineralischen Knochenzementkomponente und einer Trägerflüssigkeit die substanziell wasserfrei sind. Die offenbarten Pasten, Suspensionen oder Dispersionen besitzen eine flüssige bis pastöse Konsistenz und werden in dieser Form als Knochenzemente, oder Knochenersatzmaterialien implantiert, wobei die Abbindereaktion bevorzugt vollständig nach der Implantation erfolgt.

Lode et al. (J. Tissue Eng. Regen. Med. 2012) offenbaren, dass wasserfreie pastöse Präparationen aus mineralischen Knochenzementen über einen Druckprozess zu porösen Formkörpern gedruckt werden können, die anschließend durch Einlegen in wässrige Lösungen zu soliden Formkörpern ausgehärtet werden können (soweit in der Literatur beschrieben).

Überraschend wurde gefunden, dass so hergestellte Formkörper besonders gute mechanische Eigenschaften aufweisen, wenn die Aushärtung nicht in wässrigen Lösungen, sondern in gesättigter Wasserdampfatmosphäre bei Umgebungstemperaturen bzw. leicht erhöhten Temperaturen (25-75°C) insbesondere aber unterhalb der Sintertemperatur erfolgt.

Den bekannten Lösungsvorschlägen ist gemeinsam, dass keiner von ihnen eine verbleibende hydraulische Aktivität aufweist. Eine verbleibende hydraulische Aktivität ist vor allem aus biologischen Bedingungen wünschenswert, weil so ein Teil der Abbindereaktion der zementartigen mineralischen Präparation nach der Implantation unter biologischen Bedingungen erfolgt. Auf diese Weise kann eine höhere Bioaktivität erreicht werden.

Überdies ist eine stimulierende Wirkung auf die osteogenen Zellen wünschenswert, die durch eine direkte Patienten-individuelle Anpassung an den Knochendefekt unterstützt werden kann.

Der vorliegenden Erfindung liegt daher die Aufgabe zugrunde, formstabile Knochenersatzformkörper bereitzustellen, die unter Verzicht auf einen keramischen Sinterschritt hergestellt werden, deren Formgebung unter kontrollierten Bedingungen erfolgt, die über einen hydraulischen Abbindeprozess soweit verfestigt werden, dass eine ausreichende Formstabilität für Lagerung, Transport und die jeweiligen Implantationsbedingungen gewährleistet ist, wobei die Knochenersatzformkörper aber erst während und/oder nach der Implantation vollständig aushärten.

Erfindungsgemäß wird die Aufgabe durch formstabile Knochenersatzformkörper aus mineralischem Knochenzement mit verbleibender hydraulischer Aktivität gelöst, enthaltend
**a)** mindestens einem Anteil an abgebundenem mineralischem Knochenzement,
**b)** mindestens einen Anteil an nicht umgesetztem oder nicht abgebundenem reaktivem mineralischem Knochenzement besteht,
erhältlich durch ein Verfahren zur Herstellung formstabiler Knochenersatzformkörper mit verbleibender hydraulischer Aktivität, umfassend die folgenden Schritte:
a) Anmischen eines reaktiven mineralischen Knochenzementes mit einer schwer wasserlöslichen Trägerflüssigkeit zu einer formbaren Knochenzementmasse,
b) Formgebung der Knochenzementmasse zu einem Knochenersatzformkörper, wobei die Formgebung der Knochenzementmasse durch ein (3D)_Druckverfahren oder über einen Granulationsprozess erfolgt,
c) Kontaktieren des Knochenersatzformkörpers mit einer wässrigen Lösung oder ei-ner Wasser-(dampf-)gesättigten Umgebung, so dass ein Abbindeprozess initiiert wird und der Knochenersatzformkörper mindestens 5% des Maximalwerts der Festigkeit eines vollständig abgebundenen Knochenersatzmaterials aus den glei-chen Komponenten und mit den gleichen Strukturmerkmalen, insbesondere glei-cher Porosität, erreicht,
d) Abbruch des Abbindeprozesses durch den substantiellen Entzug von Wasser, so dass der formstabile Knochenersatzformkörper einen Anteil an abgebundenem mineralischem Knochenzement von 5 bis 90-Ma %, besonders bevorzugt von 10 bis 80-Ma%, ganz besonders bevorzugt von 30 bis 70-Ma % enthält und dass der formstabile Knochenersatzformkörper substantiell wasserfrei ist,
wobei die formstabilen Knochenersatzformkörper Druckfestigkeiten im Bereich von 2 bis 200 MPa aufweisen.

Mineralische Knochenzemente bestehen aus mindestens einer reaktiven mineralischen oder organomineralischen Feststoffkomponente (Knochenzementkomponente), die bei dem Kontaktieren mit einer wässrigen Lösung oder nach Einbringung in eine wässrige Lösung in einem hydraulischen Abbindeprozess zu einem schwerlöslichen Festkörper abbinden kann. Reaktive mineralische Knochenzemente bezeichnen hierin ausschließlich, dass die mindestens eine reaktive mineralische Knochenzementkomponente noch nicht mit Wasser zu einem abgebundenen mineralischem Knochenzement chemisch umgesetzt ist. Vorteilhaft werden die reaktiven mineralischen Knochenzementkomponenten in Form von Pulvermischungen eingesetzt, die intensiv vermischt sind, wodurch die Ausbildung eines formstabilen Knochenersatzformkörpers mit einer homogenen Zusammensetzung begünstigt wird.

Verbleibende hydraulische Aktivität bezeichnet im Sinne der Erfindung die qualitative Fähigkeit eines erfindungsgemäßen synthetischen Knochenersatzformkörpers durch die Zugabe von Wasser und/oder einer wasserhaltigen Komponente eine chemische Reaktion, d.h. einen Abbindeprozess einzugehen, der durch Wasserentzug vor Erreichen der vollständigen Umsetzung der reaktiven Komponenten unterbrochen werden kann, wobei durch den erneuten Kontakt eines erfindungsgemäßen synthetischen Knochenersatzformkörpers mit Wasser erneut eine Hydratation der verbleibenden reaktiven mineralischen Knochenzementkomponente erfolgt, wodurch der unterbrochene Abbindeprozess fortgeführt wird. Der erneute Kontakt eines erfindungsgemäßen synthetischen Knochenersatzformkörpers mit verbleibender hydraulischer Aktivität mit Wasser und/oder einer wasserhaltigen Komponente findet erst unmittelbar vor dessen Verwendung oder während oder nach seiner Verwendung, also während oder nach der Implantation in den Körper statt.

Bevorzugt weisen die formstabilen Knochenersatzformkörper mindestens 5% des Maximalwertes der Festigkeit eines vollständig abgebundenen formstabilen Knochenersatzformkörpers aus den gleichen mineralischen Knochenzementkomponenten und mit den gleichen Strukturmerkmalen, insbesondere gleicher Porosität auf. Zu diesem Zeitpunkt weisen sie eine ausreichende Formstabilität auf, um den mechanischen Belastungen, die z.B. mit Lagerung und Transport einhergehen, standhalten zu können. Vorzugsweise weisen die formstabilen Knochenersatzformkörper 15%, besonders bevorzugt 25%, ganz besonders bevorzugt 50% des Maximalwertes der Festigkeit, noch mehr bevorzugt >75% des Maximalwertes der Festigkeit eines vollständig abgebundenen formstabilen Knochenersatzformkörpers aus den gleichen mineralischen Knochenzementkomponenten und mit den gleichen Strukturmerkmalen, insbesondere gleicher Porosität auf.

Bevorzugt enthalten die formastabilen Knochenersatzformkörper, die unter biologischen Bedingungen - 37°C und Wassersättigung - umgesetzt werden können, einen Anteil an abgebundenem mineralischem Knochenzement, von 5 bis 90-Ma %, besonders bevorzugt von 10 bis 80-Ma%, ganz besonders bevorzugt von 30 bis 70-Ma %.

Die formstabilen Knochenersatzformkörper mit verbleibender hydraulischer Aktivität weisen Druckfestigkeiten von 2 bis 200 MPa, besonders bevorzugt von 5 bis 200 MPa, ganz besonders bevorzugt von 10 bis 200 MPa auf und sind damit vergleichbar mit einem Knochenersatzformkörper ohne verbleibende hydraulische Aktivität.

Die formstabilen Knochenersatzformkörper mit verbleibender hydraulischer Aktivität weisen Druckfestigkeiten im Bereich von 2 bis 100 MPa, besonders bevorzugt im Bereich von 3 bis 50 MPa, ganz besonders bevorzugt im Bereich von 5 bis 25 MPa auf und sind damit vergleichbar mit einem Knochenersatzformkörper ohne verbleibende hydraulische Aktivität.

Überraschend stellte sich heraus, dass formstabile Knochenersatzformkörper, die einen Anteil von ca. 80% mindestens einer noch nicht chemisch umgesetzten reaktiven mineralischen Knochenzementkomponente (bezogen auf den Ausgangsgehalt) enthalten, bereits 75% der maximalen Druckfestigkeit eines Knochenersatzformkörpers mit kompletter chemischer Umsetzung der mineralischen Knochenzementkomponente erreichen. Besonders überraschend stellte sich heraus, dass die formstabilen Knochenersatzformkörper, die einen Anteil von ca. 50% noch nicht chemisch umgesetzten reaktiven mineralischen Knochenzementkomponente enthalten, bereits die maximale Druckfestigkeit eines Knochenersatzformkörpers mit kompletter chemischer Umsetzung der mineralischen Komponente erreichen.

Zur Bestimmung der Druckfestigkeit der erfindungsgemäßen Knochenersatzformkörper kann eine Universalprüfmaschine wie die der Fa. Hegewald&Peschke unter Verwendung einer Kraftmesszelle von beispielsweise 20kN mit einer Vorschubgeschwindigkeit von beispielsweise 1mm/min verwendet werden. Die stoffliche Umsetzung kann in Abhängigkeit von der Aushärtedauer bei einer gewissen Temperatur und Luftfeuchtigkeit mittels Röntgendiffraktometrie (XRD) bestimmt werden. Zur Quantifizierung kann die Riedveld Methode verwendet werden. Die Rietveld-Methode dient der quantitativen Phasenanalyse, also der quantitativen Bestimmung der kristallinen Komponenten einer pulverförmigen Probe.

Bevorzugt enthält der mindestens eine Anteil an nicht umgesetztem oder nicht abgebundenem reaktivem mineralischem Knochenzement mindestens eine nicht umgesetzte oder nicht abgebundene reaktive mineralische Knochenzementkomponente mit einem Anteil von mindestens 10 Ma.-% besonders bevorzugt mindestens 20 Ma.-%, ganz besonders bevorzugt mindestens 30 Ma.-%, noch mehr bevorzugt mindestens 50 Ma.-%.

Vorteilhaft ist, dass der formstabile Knochenersatzformkörper eine hohe biologische Aktivität aufweist. Biologische Aktivität bezeichnet erfindungsgemäß die Eigenschaft des formstabilen Knochenersatzformkörpers bei der Aushärtung unter biologischen Bedingungen vorteilhaft eine dem Knochen ähnlichere Kristallstruktur zu bilden. Eine knochenähnliche nanokristalline Struktur der sich im Abbindeprozess bildenden mineralischen Strukturen ist eine wesentliche Voraussetzung für deren Interaktion mit den organischen und zellularen Elementen des sich bildenden neuen Knochens. Aus diesem Grund wird die weitestgehende Aushärtung des formstabilen Knochenersatzformkörpers nach der Implantation im Körper unter biologischen Bedingungen angestrebt. Je höher der Anteil an nicht umgesetzter oder nicht abgebundener reaktiver mineralischer Knochenzementkomponente ist, desto höher ist die biologische Aktivität. Abgestimmt auf die Anforderungen des jeweiligen klinischen Einsatzgebiets kann so ein formstabiler Knochenersatzformkörper mit entsprechender Festigkeit und biologischer Aktivität bereitgestellt werden.

Bevorzugt weisen die formstabilen Knochenersatzformkörper eine Gesamtporosität zwischen 20 und 90% auf.

Die Gesamtporosität ist die Summe der Makroporosität (makroskopisch sichtbare Poren, >ca. 100µm) und der Mikro- und Nanoporosität (nur mikroskopisch sichtbare Poren, <ca. 10µm bzw. <ca. 11µm) der formstabilen Knochenersatzformkörper. Die Makroporen, wie sie beispielsweise gedruckte formstabile 3D-Knochenersatzformkörper enthalten sind insbesondere für das Einwachsen von Zellen und Gewebestrukturen in den Formkörper bedeutsam. Die Mikro- und Nanoporen sind ein Ergebnis der Zusammensetzung des nicht umgesetzten oder nicht abgebundenen reaktiven mineralischen Knochenzements und dessen Verarbeitungsbedingungen. Sie sind entscheidend für die spezifische Oberfläche der formstabilen Knochenersatzformkörper und damit für die Absorptionsfähigkeit für biologische Moleküle und die Interaktion mit Knochenzellen.

In einer Ausführungsform liegen die formstabile Knochenersatzformkörper in Form von Granulaten vor, wobei die einzelnen Granula eine Größe von >100µm und <10mm aufweisen.

Vorteilhaft können die Granula mit biologischer Aktivität zum Auffüllen von Knochendefekten verwendet werden. Die Granula bilden bei einer weitestgehend unter biologischen Bedingungen stattfindenden Abbindereaktion nach Implantation im Körper eine dem Knochen ähnlichere Kristallstruktur.

Vorteilhaft können aus den Granula über Formgebungsprozesse (z.B. in einer Form zusammendrücken) größere Knochenersatzformkörper, angepasst an die entsprechende Defektstelle, angefertigt werden. Außerdem vorteilhaft ist die einfache Herstellbarkeit der Granula, deren hohe Bioaktivität und deren relativ hohe Druckfestigkeit.

Bevorzugt weist der formstabile Knochenersatzformkörper die Form eines gedruckten 3D-Formkörpers auf. Bevorzugt erhalten gedruckte 3D-Formkörper ihre Struktur durch ein 3D-Druckverfahren, wodurch sowohl die äußere Form, als auch die Porenstruktur in sehr weiten Bereichen vorgegeben werden kann. Bevorzugt weisen sie eine geometrische äußere Form auf, insbesondere als Zylinder oder rechteckiger Block. Bevorzugte Formen sind weiterhin Keile, Scheiben, an den Knochen anatomisch angepasste Konen oder Formen, wie sie beispielsweise von Implantaten für Cages für die Wirbelsäule bekannt sind. Bevorzugt besteht ein erfindungsgemäßer 3D-Knochenersatzformkörper aus mehreren aufeinander folgenden Schichten (gestapelt), die verschiedene Formen, wie zum Beispiel kreisförmig, oval, eckig, annehmen können. Die einzelnen Schichten bestehen aus Strängen, die so geordnet liegen, dass zwischen den Strängen Abstände entstehen, wodurch eine zusammenhängende (interkonnektierende) Makroporosität entsteht, wobei die Makroporosität im Bereich von 20 bis 90% liegt.

Vorteilhaft können mittels 3D-Druckverfahren gezielte Porenanordnungen und gezielte Porenvolumen festgelegt werden. Vorteilhaft ermöglicht es insbesondere die Gestaltung von gerichteten Poren, so dass der Weg für den einwachsenden neuen Knochen zur Überbrückung eines Knochendefekts auf ein Minimum verkürzt werden kann.

Die Aufgabe wird auch gelöst durch ein Verfahren zur Herstellung formstabiler Knochenersatzformkörper mit verbleibender hydraulischer Aktivität, umfassend die folgenden Schritte:
a) Anmischen eines reaktiven mineralischen Knochenzementes mit einer schwer wasserlöslichen Trägerflüssigkeit zu einer formbaren Knochenzementmasse,
b) Formgebung der Knochenzementmasse zu einem Knochenersatzformkörper, wobei die Formgebung der Knochenzementmasse durch ein (3D)-Druckverfahren oder über einen Granulationsprozess erfolgt,
c) Kontaktieren des Knochenersatzformkörpers mit einer wässrigen Lösung oder einer Wasser-(dampf-)gesättigten Umgebung, so dass ein Abbindeprozess initiiert wird und der Knochenersatzformkörper mindestens 5% des Maximalwerts der Festigkeit eines vollständig abgebundenen Knochenersatzmaterials aus den gleichen Komponenten und mit den gleichen Strukturmerkmalen, insbesondere gleicher Porosität, erreicht,
d) Abbruch des Abbindeprozesses durch den substantiellen Entzug von Wasser, so dass der formstabile Knochenersatzformkörper einen Anteil an abgebundenem mineralischem Knochenzement von 5 bis 90-Ma %, besonders bevorzugt von 10 bis 80-Ma%, ganz besonders bevorzugt von 30 bis 70-Ma % enthält und dass der formstabile Knochenersatzformkörper substantiell wasserfrei ist.

Bevorzugt wird der Knochenersatzformkörper durch die Entfernung von enthaltenen Hilfsstoffen, Restwasser und/oder wasserlöslichen Lösungsmitteln, die zum substantiellen Entzug des Wassers verwendet wurden, getrocknet.

Überraschenderweise hat sich gezeigt, dass durch den substantiellen Entzug von Wasser, (d.h. das vollständige Entfernen des Anteils an physikalisch gebundenem und kondensiertem Wasser) aus einem erfindungsgemäßen Knochenersatzformkörper der hydraulische Abbindeprozess und die damit einhergehende Aushärtung eines synthetischen Knochenersatzformkörpers unterbrochen werden kann. Durch das Unterbrechen des Abbindeprozesses liegt ein erfindungsgemäßer Knochenersatzformkörper in Form eines formstabilen Gefüges vor, das eine verbleibende hydraulische Aktivität aufweist.

Ein großer Vorteil besteht darin, dass die erfindungsgemäßen Knochenersatzformkörper vor dem ersten Inkontaktbringen mit Wasser oder einer Wasser-(dampf-)atmosphäre plastisch verformbar sind, aber nach dem ersten Inkontaktbringen mit Wasser oder einer Wasser-(dampf-)atmosphäre und dem vorzeitigen Unterbrechen der Abbindereaktion ein formstabiler Knochenersatzformkörper mit verbleibender hydraulischer Aktivität entsteht, dessen Härte und Sprödigkeit aber noch signifikant unter der eines vollständig ausgehärteten Knochenersatzformkörpers der gleichen mineralischen Zusammensetzung und mit den gleichen Strukturmerkmalen, insbesondere gleicher Porosität liegen kann. Der erfindungsgemäße Knochenersatzformkörper weist für den Transport, die Lagerung und die Implantation eine ausreichende mechanische Festigkeit auf. Vorteilhaft erleichtert die Formgebung und/oder Strukturierung eines erfindungsgemäßen synthetischen Knochenersatzformkörpers zu strukturierten Vorformlingen mit verbleibender hydraulischer Aktivität dem Endbenutzer (z.B. Operateur), ein Implantat auf der Basis eines erfindungsgemäßen synthetischen Knochenersatzformkörpers formschlüssig an den Defekt des Knochens anzupassen, da sich herausstellte, dass unvollständig abgebundene erfindungsgemäße Formkörper (mit verbleibender hydraulischer Aktivität) besonders gut intraoperativ nachbearbeitet werden können, bspw. durch Schnitzen mit chirurgischen Instrumenten (z. B. Skalpell), was mit handelsüblichen keramischen Formkörpern erfahrungsgemäß sehr schwierig oder unter OP-Bedingungen unmöglich ist.

Die reaktiven mineralischen Knochenzemente werden durch intensives Vermischen mindestens einer pulverförmigen reaktiven mineralischen Knochenzementkomponente mit einer Trägerflüssigkeit erhalten, so dass eine homogene formbare Knochenzementmasse in Form einer pastösen Dispersion erhalten wird.

Die pastenförmigen Dispersionen zur Herstellung des formstabilen Knochenersatzformkörpers kann verschiedenen Methoden der Formgebung unterzogen werden, wobei die Zusammensetzung der Dispersionen und ihre physikalischen Eigenschaften auf das Formgebungsverfahren abgestimmt werden. Beispielsweise werden für Gießverfahren dünnflüssigere Dispersionen verwendet als für 3D-Druckverfahren. Bevorzugte Verfahren orientieren sich an der gewünschten Form und Funktion der angestrebten Knochenersatzformkörper.

Bevorzugt erfolgt die Formgebung einer plastisch verformbaren Paste, enthaltend mindestens eine reaktive mineralische Knochenzementkomponente, die dispergiert in einer Trägerflüssigkeit ist, durch das Einwirken von Zug- und/oder Druckkräften in Form von Walzen, Drucken (z.B. Flach-, Durch-, Hoch-, Tiefdruck, gemäß DIN 16500, Siebdruck, Offsetdruck, Ink-Jet, u.a.), Extrudieren, Gießen, Rakeln, Granulieren und andere formgebende Verfahren, die dem Fachmann bekannt sind.

Ein bevorzugte Form der Knochenersatzformkörper sind Zylinder, Blöcke oder Keile. Sie können z. B. über Gießen oder Pressen in eine entsprechende Negativform erhalten werden. Diese können in der Form ausgehärtet werden (s.u.) und nach Erreichen einer ausreichenden Ausformfestigkeit aus der Form entnommen werden und nach Bedarf bis zum gewünschten Aushärtungsgrad weiter prozessiert werden.

Eine weiter bevorzugte Form der Knochenersatzformkörper sind poröse Formkörper unterschiedlicher Außengeometrien und zufallsverteilter Makroporosität. Solche Formkörper können auf unterschiedliche Weise erhalten werden, indem eine pastöse Knochenzementdispersion enthaltend einen leicht wasserlöslichen Füllstoff (z. B. Zucker, Salz, Ammoniumcarbonat, Polymerpartikel) in eine vorgegebene Form eingebracht wird. Der Füllstoff kann während oder nach der Aushärtung durch Herauslösen oder Verdampfen entfernt werden und hinterlässt dabei eine poröse Struktur. Die Formgebung kann auch nach der Aushärtung erfolgen, indem zunächst ein größerer Formkörper erzeugt wird und der gewünschte Formkörper in der Implantatgröße durch nachträgliche Bearbeitung (z. b. Fräsen, Sägen, etc.) erzeugt wird.

Eine besonders bevorzugte Form der Knochenersatzformkörper sind poröse Formkörper mit definierter und insbesondere interkonnektierender Porosität. Solche Knochenersatzformkörper sind von besonderem klinischem Interesse, da das Ziel der möglichst schnellen und vollständigen knöchernen Integration nur bei interkonnektierender Porosität zu erreichen ist und diese knöcherne Integration nur dann besonders schnell erfolgen kann, wenn die Strecke für den Knocheneinwuchs möglichst kurz ist, wenn also die Poren möglichst geradlinig und gerichtet durch den Formkörper verlaufen. Dieses Ziel ist mit dem erfindungsgemäßen Material und Verfahren insbesondere durch den 3D-Druck erreichbar.

Zur Auslösung des Abbindeprozesses wird die plastisch verformbare Paste bzw. die daraus im Formgebungsschritt hergestellten Formkörper in einem folgenden Schritt, bevorzugt graduell, mit einer wässrigen Lösung oder reinem Wasser in innigen Kontakt gebracht oder, besonders bevorzugt, in eine Wasser-(dampf)-atmosphäre eingebracht. Überraschend hat sich herausgestellt, dass sich Dispersionen aus Knochenzementpulver in einer wasserfreien Trägerflüssigkeit mit einem hohen Feststoffgehalt z. B. mittels 3D-Druck in einem Formgebungsschritt zu großen Formkörpern verarbeiten lassen, ohne ihre geometrische Form während der Herstellung und Weiterverarbeitung zu verändern. Aus diesem Grund ist es möglich, die Formkörper zunächst in beliebiger Anzahl herzustellen und sie in einem nachfolgenden Schritt gemeinsam der Auslösung des Abbindeprozesses zu unterziehen.

Bevorzugt wird der formstabile Knochenersatzformkörper so lange mit einer wässrigen Lösung oder einer Wasser-(dampf-)gesättigten Umgebung kontaktiert, so dass dieser mindestens 5%, vorzugsweise 15%, besonders bevorzugt 25%, ganz besonders bevorzugt 50%, noch mehr bevorzugt >75% des Maximalwerts der Festigkeit eines vollständig abgebundenen Knochenersatzmaterials aus den gleichen Komponenten und mit den gleichen Strukturmerkmalen, insbesondere gleicher Porosität, erreicht.

Bevorzugt wird der formstabile Knochenersatzformkörper über einen Zeitraum von 0 bis 672 h mit einer wässrigen Lösung oder einer Wasser-(dampf-)gesättigten Umgebung kontaktiert.

Bevorzugt enthält die wässrige Lösung mindestens einen Zusatzstoff ausgewählt aus einer Pufferlösung, einem organischen und/oder anorganischen Salz, einer Zellpräparation (vorzugsweise Stammzellen, Osteoprogenitorzellen), einem biologischen, rekombinanten oder pharmakologischen Wirkstoff, eine Nukleinsäure (RNA oder DNA), Mischungen von Nukleinsäuren, einer Aminosäuren, einer modifizierte Aminosäure, einem Vitamin und Mischungen aus diesen.

Vorteilhaft enthält die wässrige Lösung eine Pufferlösung, wodurch in der wässrigen Lösung ein pH-Wert fest eingestellt wird. Dem Fachmann sind verschieden Pufferlösungen bekannt. Er wird je nach einzustellenden pH-Wert eine geeignete Pufferlösung auswählen, die aus physiologisch verträglichen Komponenten besteht. Zusätzlich kann die wässrige Lösung weitere anorganische und/oder organische Salze, die selbst keine Pufferwirkung aufweisen, enthalten. Bevorzugt sind die Pufferlösung und die weiteren Salze ausgewählt aus Phosphaten, Citraten, Acetaten, Chloriden, Sulfaten und Boraten. Bevorzugte Kationen sind Alkali- und Erdalkalimetallionen, Ammoniumionen und Guanidin.

Besonders bevorzugt enthält die wässrige Lösung eine Lösung von Aminosäuren und/oder modifizierten Aminosäuren, insbesondere phosphorylierte Aminosäuren, ganz besonders bevorzugt Phosphoserin.

Vorteilhaft erfolgt so die Aushärtung unter definierten (körpernahen) biologischen Bedingungen und dennoch außerhalb des Körpers, indem der Knochenersatzformkörper vor der Implantation in einer (definierten biologischen) Lösung inkubiert wird.

Vorteilhaft werden die pharmakologischen Wirkstoffe von den Formkörpern aufgenommen und/oder adsorbiert in abhängigkeit von der Art der Wirkstoffe. Die Adsorption ist abhängig von der spezifischen Oberfläche der Formkörper. Durch die Zugabe der Wirkstoffe in der Aushärtungsphase anstatt bei den endgültig abgebundenen Formkörpern wird ein effektiveres und praktikableres Verfahren bereitgestellt. Einige bevorzugte Wirkstoffe (biologische und pharmakologische) beeinflussen die Nanostruktur und damit die spezifische Oberfläche der Formkörper vorteilhaft. Sie werden einerseits spezifisch gebunden und führen andererseits zu einer feineren Nanostruktur, was u. a. die mechanischen Eigenschaften verbessert, die Resorbierbarkeit erhöht und im Falle von Wirkstoffen zusätzlich die Abgabe steuert.

Unter Wasser, das substanziell entzogen werden kann, wird erfindungsgemäß physikalisch gebundenes oder kondensiertes Wasser verstanden. Bevorzugt erfolgt der substantielle Entzug von Wasser durch die Variation physikalischer Umgebungsparameter (z.B. Verringerung des Drucks und/oder Erhöhung der Temperatur oder Gefriertrocknung) und/oder durch das Kontaktieren mit einem wasserlöslichen Lösungsmittel, bevorzugt kurzkettiger Alkohole wie Methanol, Ethanol, Isopropanol und/oder Propanol. Ketone, z.B. Aceton, Lactone wie γ-Butyrolacton, Lactame wie N-Methyl-2-pyrrolidon, Nitrile wie Acetonitril, Nitroverbindungen wie Nitromethan, tertiäre Carbonsäureamide wie Dimethylformamid, Harnstoffderivate wie Tetramethylharnstoff oder Dimethylpropylenharnstoff (DMPU), Sulfoxide wie Dimethylsulfoxid (DMSO), Sulfone wie Sulfolan, Kohlensäureester wie Dimethylcarbonat oder Ethylencarbonat, können ebenfalls zum Entzug von Wasser eingesetzt werden.

Im Unterschied zum substantiellen Entzug von Wasser durch die Variation physikalischer Umgebungsparameter (z.B. Verringerung des Drucks und/oder Erhöhung der Temperatur oder Gefriertrocknung) oder durch das Kontaktieren des synthetischen Knochenersatzmaterials mit einem wasserlöslichen Lösungsmittel (z.B. Lösungsmittelaustausch von Wasser gegen Aceton) kann der substantielle Entzug von Wasser auch durch dessen Verbrauch in der chemischen Abbindereaktion erfolgen. In diesem Fall genügt die vollständige Umwandlung des vorhandenen Anteils an physikalisch gebundenem Wasser in chemisch gebundenes Wasser, wobei definiert weniger Wasser zur Verfügung gestellt wird, als zur vollständigen Umsetzung innerhalb einer vollständigen Abbindereaktion erforderlich wäre.

Bevorzugt wird durch das Kontaktieren des formstabilen Knochersatzformkörpers mit einem wasserlöslichen Lösemittel nicht nur Wasser substantiell entfernt, sondern je nach der Art des eingesetzten wasserlöslichen Lösemittels werden auch die Trägerflüssigkeit und andere Hilfsstoffe ausgewaschen. Bevorzugt wird der Fachmann das wasserlösliche Lösemittel entsprechend der Löslichkeitseigenschaften der eingesetzten Trägerflüssigkeit und Hilfsstoffe auswählen. Bevorzugt werden die Trägerflüssigkeit und die Hilfsstoffe vollständig aus dem formstabilen Knochenersatzformkörper ausgewaschen. Ein teilweiser Verbleib der Trägerflüssigkeit und/oder der Hilfsstoffe im erfindungsgemäßen formstabilen Knochenersatzformkörper wird dadurch nicht ausgeschlossen. Durch den Einsatz biokompatibler Stoffe als Trägerflüssigkeit und Hilfsstoffe wird auch der Einsatz derartiger formstabiler Knochenersatzformkörper im menschlichen Körper ermöglicht.

Bevorzugt wird das Wasser substantiell entzogen, wenn der formastabile Knochenersatzformkörper einen Anteil an abgebundenem mineralischem Knochenzement, von 5 bis 90-Ma %, besonders bevorzugt von 10 bis 80-Ma%, ganz besonders bevorzugt von 30 bis 70-Ma % enthält.

Der formstabile Knochenersatzformkörper wird so lange mit wasserlöslichem Lösemittel kontaktiert bzw. gewaschen bis der formstabile Knochenersatzformkörper substantiell wasserfrei ist. Nach einer besonderen Ausgestaltung der Erfindung wird der formstabile Knochenersatzformkörper nacheinander mit verschiedenen Lösemittel(-gemischen) kontaktiert bzw. gewaschen.

Der erhaltene Formkörper mit verbleibender hydraulischer Aktivität kann nach dem substantiellem Entzug von Wasser durch die Variation physikalischer Umgebungsparameter (z.B. Erhöhung der Temperatur und/oder Verringerung des Druckes, Gefriertrocknung) getrocknet werden, um enthaltene Hilfsstoffe, Restwasser und wasserlösliche Lösungsmittel, die zum substantiellen Entzug von Wasser verwendet wurden, zu entfernen.

Grundsätzlich kann die Entwässerung, Waschung und Trocknung auch unter anderen Bedingungen und mit anderen Lösungsmitteln erfolgen. Dem Fachmann wird offensichtlich sein, in Abhängigkeit von den ausgewählten Trägerflüssigkeiten und Hilfsstoffen, sowie angemessen für den geplanten Einsatz ein geeignetes Lösungsmittel für die Entwässerung und Waschung der Formkörper auszuwählen. Die geeigneten Waschungs- und Trocknungsbedingungen ergeben sich ebenfalls aus technischen Überlegungen zu den gewählten Hilfsstoffen (inkl. sicherheitstechnischen und arbeitsschutztechnischen Überlegungen) und sind dem Fachmann geläufig.

In einer besonderen Ausgestaltung der Erfindung wird die Abbindereaktion der Formkörper in mehreren Schritten und/oder mit verschiedenen wässrigen Lösungen und/oder Wasserdampfatmosphäre durchgeführt. Dazu wird zunächst der mineralische Knochenzement einem Formgebungsprozess unterzogen, indem die Form des Knochenimplantats grob vorgegeben wird oder bereits die Endform (net-shape) festgelegt wird. Anschließend erfolgt der Abbindeprozesses des Formkörpers zu einem formstabilen Knochenersatzformkörper durch Kontaktieren mit einer wässrigen Lösung und/oder einer Wasserdampfatmosphäre. Der Abbindeprozess wird zu einem festgelegten Zeitpunkt (bzw. nach Erreichen eines definierten Umsetzungsgrades) - vor der vollständigen Umsetzung - durch den erfindungsgemäßen Entzug von Wasser unterbrochen. Zu einem späteren Zeitpunkt wird der Abbindeprozess durch erneutes Kontaktieren mit einer wasser- oder wasserdampfhaltigen Umgebung fortgesetzt. Dieses Vorgehen ermöglicht die Durchführung des Abbindeprozesses in sukzessiven Schritten unter Veränderung der Reaktionsbedingungen, insbesondere in unterschiedlichen wässrigen Lösungen. Vorteilhaft kann somit die Abbindereaktion unter verschiedenen und/oder definierten Bedingungen durchgeführt werden. Dazu kann die Formstabilität des Formkörpers in einem ersten Schritt erreicht werden. Die Unterbrechung und Wiederaufnahme des Abbindeprozesses kann beliebig oft erfolgen. In einer letzten Abbindereaktion kann der formstabile Knochenersatzformkörper schließlich vollständig abgebunden werden. Insbesondere kann der Hersteller des Formkörpers in einem ersten Schritt einen geringgradig abgebundenen Knochenersatzformkörper erzeugen. Ein Weiterverarbeiter kann ausgehend von diesem Vorprodukt die teilweise oder vollständige Aushärtung beispielsweise in Gegenwart von definierten biologischen (Zellen, Geweben), rekombinanten und/oder pharmakologischen Wirkstoffen durchführen und damit das Endprodukt herstellen.

Erfindungsgemäß ist der formstabiler Knochenersatzformkörper sowohl ein geringgradig abgebundener Knochenersatzformkörper, als auch ein teilweise oder vollständig abgebundener Knochenersatzformkörper, sofern dieser vor der vollständigen Abbindereaktion durch Wasserentzug in einem Zwischenschritt in einen formstabilen Zustand bzw. Formkörper überführt wurde, in dem eine verbleibende hydraulische Aktivität vorlag.

Geringgradig abgebunden bezeichnet im Sinne der Erfindung einen formstabilen Formkörper, dessen Abbindereaktion zu einem frühen Zeitpunkt unterbrochen wurde, insbesondere zu einem Zeitpunkt, an dem weniger als 30% der hydraulisch aktiven Bestandteile umgesetzt sind. Der Zeitpunkt wird bevorzugt so gewählt, dass der Formkörper ohne Beschädigung weiterverarbeitet werden kann.

Bevorzugt wird als mindestens eine reaktive mineralische Knochenzementkomponente zum Anmischen des reaktiven mineralischen Knochenzementes eine Substanz aus der Gruppe der Silikate, Phosphate, Sulfate, Carbonate, Oxide und/oder Hydroxide in Verbindung mit Calciumionen, Magnesiumionen und/oder Strontiumionen eingesetzt, die bei dem Kontaktieren mit einer wässrigen Lösung oder nach Einbringung in eine wässrige Lösung in einem hydraulischen Abbindeprozess zu einem schwerlöslichen Festkörper abbinden kann. Besonders bevorzugt sind Knochenzementkomponenten, die zu Calciumphosphaten und/oder Magnesiumphosphaten abbinden.

Bevorzugt enthält die reaktive mineralische Knochenzementkomponente Calcium- und/oder Magnesiumsalze der Ortho-Phosphorsäure.

Besonders bevorzugt sind ebenfalls reaktive mineralische Knochenzementkomponenten, enthaltend Mischungen aus Calciumphosphaten und/oder Magnesiumphosphaten mit Carbonaten, Oxiden oder Hydroxiden des Calciums, Magnesiums oder Strontiums.

Besonders bevorzugt sind ebenfalls reaktive mineralische Knochenzementkomponenten enthaltend Mischungen aus Carbonaten, Oxiden oder Hydroxiden des Calciums, Magnesiums oder Strontiums mit Alkaliphosphaten (mono-, di- und tri-Alkaliphosphaten), mono- und di-Ammoniumphosphaten oder Alkalisilikaten.

Bevorzugt wird der reaktive mineralische Knochenzement mit einer Trägerflüssigkeit zu einer formbaren Knochenzementmasse angemischt. Bevorzugt ist die Trägerflüssigkeit eine organische wasserfreie Substanz, in der die mindestens eine reaktive mineralische Knochenzementkomponente dispergiert ist.

Bevorzugt ist die organische Trägerflüssigkeit so ausgewählt, dass sie selbst nicht mit der mindestens einen pulverförmigen reaktiven mineralischen Knochenzementkomponente reagiert. Grundsätzlich sind sowohl wasserlösliche als auch schwer wasserlösliche als auch wasserunlösliche organische Trägerflüssigkeiten geeignet. Unter schwer wasserlöslich werden im Sinne der Erfindung organische Trägerflüssigkeiten verstanden, deren maximale Löslichkeit in Wasser 1,0 mol/l, bevorzugt 0,1 mol/l, beträgt. Organische Trägerflüssigkeiten mit einer maximalen Löslichkeit in Wasser von mehr als 1 mol/l (vorzugsweise mehr als 3 mol/l) werden hierin als wasserlöslich bezeichnet. Bevorzugt sind jedoch schwer wasserlösliche Trägerflüssigkeiten. Besonders bevorzugt sind hydrophobe, praktisch wasserunlösliche Trägerflüssigkeiten. Die in der wasserfreien Zubereitung enthaltene Trägerflüssigkeit ist vorzugsweise bioverträglich.

Bevorzugt werden beim Anmischen des reaktiven mineralischen Knochenzements zu einer formbaren Knochenzementmasse Zusatzstoffe verwendet. Zusatzstoffe sind beispielsweise oberflächenaktive Substanzen (Tenside), pharmakologische Wirkstoffe und Füllstoffe.

In einer bevorzugten Ausgestaltung der Erfindung enthält die Trägerflüssigkeit der Dispersion der mindestens einen reaktiven mineralischen Knochenzementkomponente zur Herstellung eines synthetischen Knochenersatzformkörpers oberflächenaktive Substanzen, die das Eindringen von Wasser oder Wasserdampf oder Luftfeuchtigkeit zur Auslösung und zum Fortschreiten der Abbindereaktion unterstützen oder ermöglichen, das gilt insbesondere bei der Verwendung hydrophober Trägerflüssigkeiten. Bevorzugt sind die oberflächenaktiven Substanzen ausgewählt aus der Gruppe der Tenside, besonders bevorzugt aus der Gruppe nichtionischer und anionischer Tenside.

Ein weiterer Bestandteil der formbaren Knochenzementmasse ist vorzugsweise mindestens ein Abbindebeschleuniger. Dadurch wird vorteilhaft die Abbindekinetik bei der Aushärtung der erfindungsgemäßen Dispersion eingestellt und kontrolliert. Bevorzugte Abbindebeschleuniger sind Phosphatsalze, organische Säuren oder Salze organischer Säuren. Bevorzugt sind Natrium- und/oder Kaliumionenhaltige oder Ammoniumionen-haltige Phosphate oder Natrium- und/oder Kaliumionenhaltige oder Ammoniumionen-haltige Salze organischer Säuren und deren Mischungen untereinander.

In einer besonderen Ausgestaltung der Erfindung ist der Abbindebeschleuniger Bestandteil der wässrigen Lösung, die den Abbindeprozess initiiert.

Außerdem können pharmakologische Wirkstoffe in die formbaren Knochenzementmasse eingearbeitet werden. Bevorzugt sind pharmazeutische Wirkstoffe mit einer (knochen-) wachstumsstimulierenden oder antimikrobiellen Wirkung. Besonders bevorzugte Wirkstoffe sind ausgewählt aus Antibiotika, Antiseptika, antimikrobiellen Peptiden, Nukleinsäuren (vorzugsweise siRNA), antiresorptiven Wirkstoffen (vorzugsweise Bisphosphonaten, Cortikoiden, Fluoriden, Protonenpumpen-Inhibitoren), PTH und dessen Derivate, knochenwachstumsstimulierenden Wirkstoffen vorzugsweise Wachstumsfaktoren, Vitamine, Hormone, Morphogene, davon bevorzugt knochenmorphogenetische Proteine und Peptide, sowie angiogene Wirkstoffe und unter diesen besonders bevorzugt Fibroblasten Wachstumsfaktoren (aFGF, bFGF, FGF18 etc.), Entzündungshemmer und Antitumorsubstanzen.

Vorteilhaft üben die Nukleinsäuren (insbesondere die siRNA) eine regulatorische Wirkung auf Zellen in der Umgebung des implantierten Formkörpers aus.

Die erfindungsgemäßen synthetischen Knochenersatzformkörper eignen sich besonders gut als Träger für pharmakologische Wirkstoffe. Einerseits liegt dies daran, dass die Bereitstellung des erfindungsgemäßen synthetischen Knochenersatzformkörpers bei niedrigen Temperaturen (vorzugsweise unter 80°C, besonders bevorzugt <60°C, weiter bevorzugt <=37°C) erfolgt. Dies erlaubt die problemlose Einarbeitung von temperatursensitiven Wirkstoffen. Andererseits bietet das erfindungsgemäße Verfahren zur Herstellung synthetischer Knochenersatzformkörper eine verbesserte Wirkstofffreisetzung, da die Wirkstoffe durch die Resorption des mineralischen Knochenzements sukzessive freigesetzt werden. Damit erlaubt das erfindungsgemäße Verfahren zur Herstellung formstabiler Knochenersatzformkörper eine kontrollierte Wirkstofffreisetzung in einem weiten Bereich und ist für den Einsatz temperatursensitiver Wirkstoffe in besonders vorteilhafter Weise geeignet.

Weitere bevorzugte Bestandteile der formbaren Knochenzementmasse, vorzugsweise der wasserfreien Zubereitung, sind Füllstoffe. Bevorzugt sind wasserlösliche partikuläre Füllstoffe aus mineralischen oder organischen Substanzen. Durch den Einsatz wasserlöslicher Partikel kann vorteilhaft die Porosität des bei Aushärtung mit Wasser gebildeten Feststoffs eingestellt werden. Wasserlösliche Füllstoffe weisen vorzugsweise eine Partikelgröße von 10 µm bis 2000 µm, bevorzugt von 100 µm bis 1000 µm, auf. Wasserlösliche Füllstoffe sind in der wasserfreien Zubereitung bevorzugt zu 5 bis 90 Vol.-%, vorzugsweise 10 bis 80 Vol.-% enthalten (bezogen auf das Gesamtvolumen der wasserfreien Zubereitung/Dispersion). Bevorzugte wasserlösliche Füllstoffe sind ausgewählt aus Zuckern (vorzugsweise Saccharose), Zuckeralkoholen (vorzugsweise Sorbit, Xylit, Mannit), wasserlöslichen Salzen (vorzugsweise Natriumchlorid, Natriumcarbonat, Ammoniumcarbonat oder Calciumchlorid). Der Porenanteil des ausgehärteten mineralischen Knochenzements beträgt bevorzugt 10 bis 90 Vol.-%, besonders bevorzugt 10 bis 75 Vol.-%. Die Poren weisen bevorzugt eine mittlere Porenweite (maximale innere Ausdehnung einer Pore) von 100 - 2000 µm, besonders bevorzugt 100 - 1000 µm auf.

Besonders bevorzugt sind sämtliche Bestandteile des formstabilen Knochenersatzformkörpers im Körper resorbierbar. Besonders bevorzugt für die Erfindung ist die Kombination von resorbierbaren metallischen oder mineralischen Fasern und dem reaktiven mineralischen Knochenzement zur Herstellung der erfindungsgemäßen synthetischen Knochenersatzformkörper zur Herstellung von Faserverbundstoffen.

Optional enthält die formbare Knochenzementmasse polymere Zusätze, vorzugsweise ausgewählt aus Chitosan, Hyaluronsäure, Gelatine, Kollagen, Chondroitinsulfat, Cellulosederivate, Stärkederivate, Alginat, wasserlösliche Acrylate, Polyethylenglycol, Polyethylenoxid, PEG-PPG-Copolymeren, Polyvinylpyrrolidon und Copolymeren aus wasserlöslichen Acrylaten mit Polyethylenglycol und/oder Polyethylenoxid.

Wie bereits zuvor erwähnt, enthalten handelsübliche Zemente zuweilen große Mengen an Füllstoffen, die nur oberflächlich an der Reaktion teilnehmen (also insbesondere in die Zementmatrix eingebunden werden), aber nur mittelbar an dem Abbindeprozess beteiligt sind. Die Teilnahme kann beispielsweise auch darin bestehen, dass ein Füllstoff als Kristallisationskeim für die Mineralisation während des Abbindeprozesses fungiert und damit insbesondere die Abbindekinetik beeinflusst, aber selbst nicht umgesetzt wird. Dies gilt beispielsweise im Falle der Calciumphosphat-Zemente für den Zusatz an gefälltem Hydroxylapatit. Für technische Zemente werden kostengünstige Zusätze vielfach auch aus ökonomischen Gründen zur Streckung zugesetzt.

Bevorzugt erfolgt die Initiation des Abbindeprozesses in einer gesättigten Wasserdampfatmosphäre bei > 90% relativer Luftfeuchtigkeit, vorteilhaft kontrolliert durch die portionsweise Zufuhr einer definierten Menge von Wasserteilchen, bei einer Temperatur zwischen 0 und 100°C, besonders bevorzugt zwischen 25 und 75°C.

Überraschenderweise wurde gefunden, dass der bevorzugte Temperaturbereich deutlich unterhalb der Sintertemperatur liegt und eine Erhöhung der Temperatur weder mit einer schnelleren Abbindereaktion, noch mit einem schnelleren Anstieg der Druckfestigkeit verbunden ist.

Bevorzugt erfolgt der Abbindeprozess in einer Wasser-(dampf-)atmosphäre bei > 90% relativer Luftfeuchtigkeit und einer Temperatur zwischen 0 und 100°C.

Vorteilhaft kann der Abbindeprozess in mehreren Stufen erfolgen, indem beispielsweise zunächst, nach dem Schritt der Formgebung, der Abbindeprozess bei hoher Luftfeuchtigkeit - > 90% - initiiert wird und anschließend ein oder mehrere weitere(r) Aushärteschritt(e) unter abweichenden Bedingungen erfolgen. In diesem Fall kann einer der Aushärteschritte auch in wässriger Lösung erfolgen. Einer der Aushärteschritte (bevorzugt der abschließende) kann auch unter erhöhter Temperatur (>100°C) und unter erhöhtem Druck (>1 bar) im Autoklaven erfolgen und so gleichzeitig der Sterilisation der Formkörper dienen. Ebenso ist es möglich einen oder mehrere der Abbindeschritte nach der Unterbrechung des Abbindeprozesses durch Entzug des Wassers und der Waschung der Formkörper (und anschließenden Trocknung) durchzuführen. Auch in diesem Fall ist es vorteilhaft, den letzten Abbindeschritt als Autoklavierung ggf. mit gleichzeitiger Sterilisation vorteilhaft in der endgültigen Verpackung durchzuführen. Entscheidend ist, dass nach der Durchführung aller Abbindeschritte im erfindungsgemäßen endgültigen Knochenersatzformkörper hydraulische Restaktivität erhalten ist.

Bevorzugt erfolgt der Abbruch des Abbindeprozesses durch den substantiellen Entzug von Wasser bei einer Temperatur zwischen 0 und 100°C. Bevorzugt erfolgt der substantielle Entzug von Wasser durch das Kontaktieren des formstabilen Knochenersatzformkörpers mit einem wässrigen Lösungsmittel.

Bevorzugt erfolgt die Formgebung der Knochenzementmasse durch ein (3D-) Druckverfahren. Bevorzugt wird eine Paste, enthaltend reaktive mineralische Knochenzementkomponenten in einer wasserfreien Trägerflüssigkeit, in Form von Strängen extrudiert und geordnet und in aufeinander folgenden Schichten (gestapelt) abgelegt, so dass zwischen den Strängen Abstände verbleiben und durch die Anordnung der Schichten ein 3D-Formkörper mit zusammenhängender (interkonnektierender) Porosität entsteht und die so erhaltenen formstabilen Knochenersatzformkörper eine Gesamtporosität zwischen 20 und 90% aufweisen.

Das 3D-Druckverfahren kann beispielsweise so erfolgen (ohne im Prinzip durch die vorliegende Beschreibung eingeschränkt zu werden), dass eine vorher beschriebene Dispersion als Paste, enthaltend reaktives mineralisches Knochenzementpulver in einer wasserfreien Trägerflüssigkeit, in Form von dünnen (z. B. 0,3-0,6 mm Ø) Strängen extrudiert und geordnet und in aufeinander folgenden Schichten (gestapelt) abgelegt wird, wobei die aufeinanderfolgenden Schichten um einen vorgegebenen Winkel zwischen 0° und 90° versetzt angeordnet sind, so dass zwischen den Strängen Abstände verbleiben und durch die Anordnung der Schichten ein 3D-Formkörper mit zusammenhängender (interkonnektierender) Porosität entsteht und die so erhaltenen formstabilen Knochenersatzformkörper eine Gesamtporosität zwischen 20 und 90% aufweisen. Mit diesem Verfahren können Formkörper mit fast beliebiger Dimension und Porenanordnung gedruckt werden. Überraschend hat sich herausgestellt, dass mit diesem Verfahren und der anschließenden Aushärtung der gedruckten Formkörper in einer wasserdampfgesättigten Atmosphäre eine hohe Maßhaltigkeit der Formkörper erreicht wird und weder Strangbrüche noch Oberflächenveränderungen an den Einzelsträngen auftraten (mit den meisten beschriebenen sonstigen 3D-Druckverfahren für keramische Formkörper als Knochenimplantate werden sowohl Schwindung, als auch sonstige Beschädigungen im Herstellprozess beschrieben; und insbesondere deutlich engere Beschränkungen bei geringen Stegdicken und kleinen Porenabmessungen).

Bevorzugt erfolgt die vollständige Aushärtung des synthetischen Knochenersatzformkörpers mit verbleibender hydraulischer Aktivität oder eines Vorformlings - nach der ersten Teilaushärtung und Entwässerung - durch erneutes Kontaktieren mit einer wässrigen Flüssigkeit, enthaltend biologische Komponenten und/oder pharmakologische Wirkstoffe und/oder isolierte oder kultivierte Zellen.

Bevorzugt erfolgt die Formgebung der Knochenzementmasse über einen Granulationsprozess bei dem die formstabilen Knochenersatzformkörper in Form von Granulaten bestehend aus einzelnen Granula oder Agglomeraten aus Granula erhalten werden. Die Granulatherstellung erfolgt in bekannter Weise, wie zum Beispiel die Wirbelschichtgranulation oder die Granulation mittels eines Extrusionsprozesses. Bei der Granulation mittels Extrusionsprozess wird die pastenförmige Dispersion bestehend aus einem reaktiven mineralischen Zementpulver und einer wasserfreien Trägerflüssigkeit aus einer Kartusche über eine kalibrierte Auslassöffnung gepresst. Der extrudierte Strang wird in kurzen Abständen abgeschert und auf diese Weise werden zylinderförmige Abschnitte der Paste erhalten, die anschließend auf einem Granulierteller abgerundet werden können. Die so erhaltenen Granula können teilweise ausgehärtet und verfestigt werden.

Die Erfindung betrifft auch die Verwendung eines formstabilen Knochenersatzformkörpers zur Herstellung eines alloplastischen Implantats.

Verwendung eines formstabilen Formkörpers als Trägermaterial in der Zellkultur, der Gewebekultur und/oder des Tissue Engineering.

Erfindungsgemäße Formkörper mit hydraulischer Restaktivität können vielseitig verwendet werden, insbesondere als Zellkulturträger für die Kultivierung von Knochenzellen in der Forschung oder für das Tissue Engineering. Weitere bevorzugte Anwendungen sind als Trägermaterialien in der Biotechnologie, wobei z. B. die kultivierten Bakterien- oder Hefezellen bereits im Herstellprozess in das Trägermaterial eingebracht werden können. Eine weitere bevorzugte Anwendung ist die Reinigung von (Ab-) Wasser von Schwermetallen und organischen Substanzen.

Besonders bevorzugt ist die Verwendung als alloplastisches Implantatmaterial für die Auffüllung von Knochendefekten, die angeboren sein können, als Folge von Unfällen auftreten können, nach chirurgischen Eingriffen - insbesondere nach Entfernung von Knochentumoren, Zysten, Prothesenwechseln, Umstellungsosteotomien, etc. - auftreten können. Besonders bevorzugt ist die Verwendung der erfindungsgemäßen Knochenersatzformkörper in der Mund-/Kieferchirurgie zum Aufbau neuen Knochens, z. B. Kieferkammaufbau, Sinuslift oder Füllung von Extraktionshöhlen.

Weiterhin besonders bevorzugt ist die Verwendung der erfindungsgemäßen Knochenersatzformkörper in der Orthopädie, Unfallchirurgie und Wirbelsäulenchirurgie zur Auffüllung von Knochendefekten aller Art. Gegenüber anderen derzeit verwendeten biologischen und alloplastischen Knochenersatzmaterialien bieten die erfindungsgemäßen Knochenersatzformkörper den Vorteil der definierten Porosität, der erhöhten Bioaktivität, der definierten mechanischen Eigenschaften, der einfachen intra-operativen Bearbeitbarkeit und der guten Kombinierbarkeit mit biologischen Substanzen, Knochenzellen und pharmakologischen Wirkstoffen.

Besonders bevorzugt ist die Verwendung der erfindungsgemäßen Knochenersatzformkörper im Rahmen des therapeutischen Tissue Engineering. Hierzu werden erfindungsgemäße Knochenersatzformkörper in vitro mit Knochenzellen (bevorzugt autologe Knochenzellen oder Stammzellen) unter sterilen Bedingungen inkubiert, wobei die formstabilen Knochenersatzformkörper mit den Zellen besiedelt werden und die Zellen vermehrt werden können. Nach Abschluss der Kultivierung wird ein autologisiertes Knochenersatzimplantat erhalten, das insbesondere bei der Regeneration großer Knochendefekte indiziert ist.

Die Kombination der Formkörper mit Knochenzellen oder Stammzellen erfolgt bevorzugt in einem Bioreaktor, wobei die erfindungsgemäßen Formkörper hierfür besonders gute Voraussetzungen mitbringen, insbesondere - neben den oben genannten Eigenschaften - die variable Formgebung ohne technologische Größenbeschränkung bei gleichzeitig vollständiger interkonnektierender Porosität, die eine entscheidende Voraussetzung für die gute Perfusion mit Kulturmedium im Bioreaktor ist.

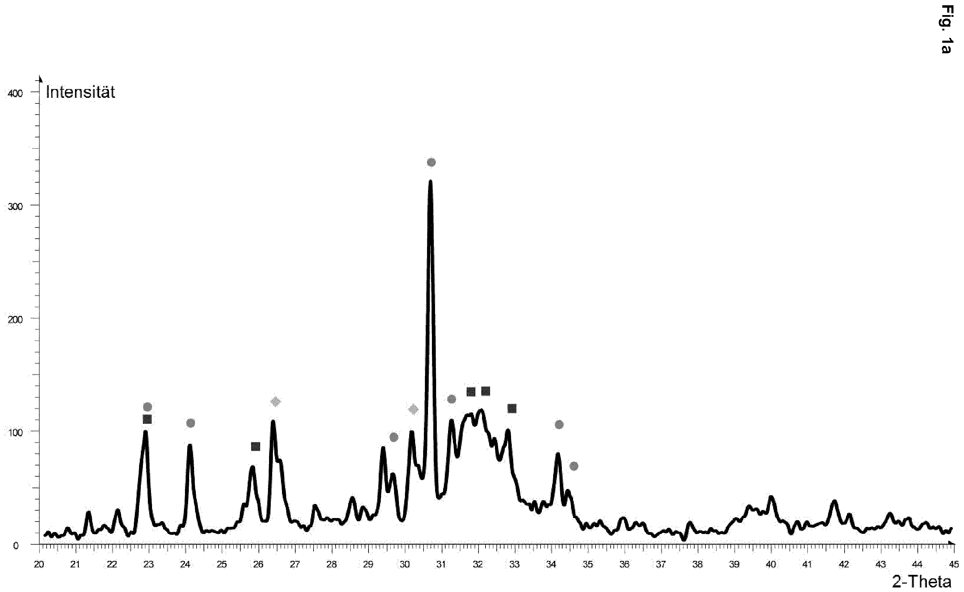

Anhand der aufgeführten Darstellungen und Ausführungsbeispiele soll die Erfindung näher erläutert werden ohne sie auf diese zu beschränken, dabei zeigen
- **Fig. 1a)**: Röntgendiffraktogramm eines porösen gedruckten Formkörpers aus Pasten-CPC, der für 2 Tage unter Wasserdampfsättigung bei 50°C ausgehärtet und dessen Aushärtung anschließend durch Entwässerung mit Aceton (3x 20 min. Acetonwaschung im Verhältnis 1:5 w/w) und Trocknung bei 80°C gestoppt wurde. Die Reflexe zeigen als vorherrschende kristalline Phase α-TCP (●) und einen breiten Reflex für nanokristallines Hydroxylapatit (■), das als Reaktionsprodukt der hydraulischen Abbindereaktion entsteht. Ein weiterer reaktiver Bestandteil ist das Monetit (◆), dessen Reflexe noch deutlich zu erkennen sind. Die Reaktion wurde vor der vollständigen hydraulischen Abbindung gestoppt.

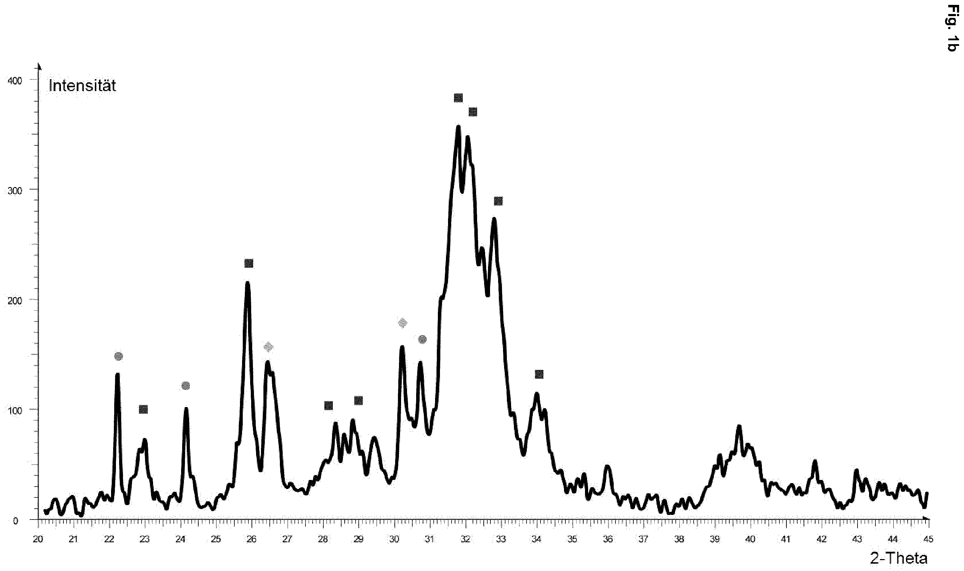

- **Fig. 1b)**: Röntgendiffraktogramm eines porösen gedruckten Formkörpers aus Pasten-CPC, der für 14 Tage unter Wasserdampfsättigung bei 50°C ausgehärtet und dessen Aushärtung anschließend durch Entwässerung mit Aceton (3x 20 min. Acetonwaschung im Verhältnis 1:5 w/w) und Trocknung bei 80°C gestoppt wurde. Die Reflexe zeigen die Anwesenheit von nur noch geringen Mengen an α-TCP (●) und Monetit (◆), als Zeichen für eine fast vollständig abgeschlossene hydraulische Umsetzung. Die dominierende Phase ist nanokristallines Hydroxylapatit (■), das durch den breiten Reflex zwischen 31,5 und 33,5° charakterisiert ist.

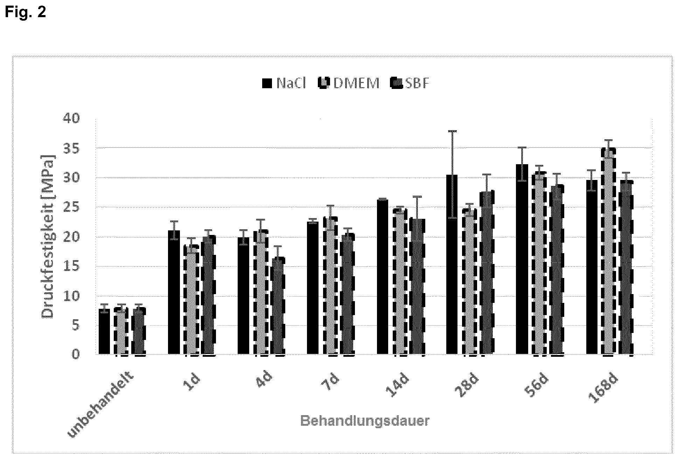

- **Fig. 2)**: Festigkeitsverlauf nach unterschiedlichen Inkubationszeiten in unterschiedlichen wässrigen Medien der nach Ausführungsbeispiel 1.6 hergestellten formstabilen Knochenersatzform körper.

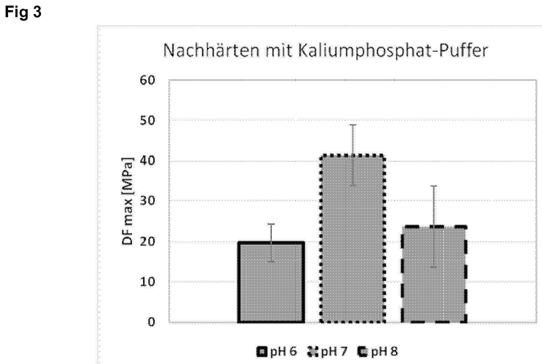

- **Fig. 3)**: Druckfestigkeit (DF) der Formkörper in Abhängigkeit vom pH-Wert der Inkubationslösung.

### Ausführungsbeispiel 1

### 1.1 Anmischen eines reaktiven mineralischen Knochenzementes zu einer formbaren Knochenzementmasse

Es wurde ein Calciumphosphatzement-Pulver auf der Basis von α-TCP hergestellt, indem 60 g α-Tricalciumphosphat, 26 g Calciumhydrogenphosphat (wasserfrei), 10 g Calciumcarbonat und 4 g Hydroxylapatit gemischt und anschließend fein vermahlen wurden. Zu 82 g dieser Pulvermischung wurden 2,5 g gemahlenes di-Kaliumhydrogenphosphat zugesetzt und anschließend in 15,5g einer Öl-Emulgatormischung bestehend aus kurzkettigen Triglyceriden (Milyol 812), Castorölethoxylat (Cremophor ELP) und Cetylphosphat (Amphisol A) (Mengenverhältnis w/w 82:13:5) dispergiert. Die erhaltene Mischung wurde zu einer plastisch verformbaren Paste vermengt und vermahlen. Nach Abschluss des Mahlvorgangs wurde die Paste in handelsübliche 5 ml PE-Kartuschen abgefüllt und bis zur weiteren Verwendung gelagert.

### 1.2 Formgebung

Die mit der beschriebenen Paste befüllten 5 ml PE Kartuschen wurden über ihre Luer-Lock-Verbindung mit einer Edelstahlkanüle mit 0,3 mm Innendurchmesser verbunden und in einen handelsüblichen 3D-Drucker (regenHU, Schweiz) montiert. Mittels Druckluft wurde ein Strang der plastisch verformbaren Paste durch die Kanüle gedrückt und nach einem computergesteuerten System auf einer Glasplatte lagenweise abgelegt. Durch die Wahl des Strang-abstands wurde die Porosität der gedruckten Formkörper vorgegeben. Die aufeinanderfolgenden Lagen waren um einen Winkel von 90° versetzt. Die Konsistenz der beschriebenen Paste ermöglichte das Drucken von Formkörpern mit einer Höhe von 15 mm, ohne erkennbare Verformung der unteren Stränge. Im gewählten Beispiel wurden Formkörper der Dimension 10x10x5mm hergestellt. Die Gesamtporosität betrug ca. 60%.

### 1.3 Aushärtung der Formkörper

Nach Abschluss des Druckvorgangs wurde die Glasplatte mit dem gedruckten Formkörper in eine Petrischale gelegt. Die Petrischale wurde in einen wasserdichten Folienbeutel gelegt, der zusätzlich einen wassergesättigten Schwamm enthielt. Der Folienbeutel wurde fest verschlossen. Der wassergesättigte Schwamm und der gedruckte Formkörper hatten keinen direkten Kontakt. Die so verpackten Formkörper wurden in einem Brutschrank bei 50°C für unterschiedliche Zeiträume inkubiert.

### 1.4 Abbruch der Abbindereaktion

Zum Abbruch der Abbindereaktion wurden die Formkörper zu unterschiedliche Zeitpunkten aus dem Brutschrank entnommen und für 20 min. in ein Glasgefäß mit wasserfreiem Aceton eingelegt (Verhältnis Aceton zu Formkörper: 5:1 (w/w)) und leicht geschüttelt. Der Vorgang wurde 3x wiederholt um sowohl Wasser, als auch das Öl-Emulgatorgemisch zu entfernen. Nach der letzten Waschung in Aceton wurden die Formkörper bei 80°C im Trockenschrank getrocknet und anschließend luftdicht in Folienbeutel verpackt.

### 1.5 Analyse der formstabilen Formkörper hinsichtlich ihrer Druckfestigkeit und Stoffumsetzung in Abhängigkeit von der Dauer der Abbindereaktion

Die nachfolgende Tabelle zeigt die Druckfestigkeit der hergestellten Formkörper in Abhängigkeit von der Aushärtedauer bei 50°C und gesättigter Luftfeuchtigkeit. Die Druckfestigkeit wurde an Formkörpern der Dimension 10x10x5mm (liegend) bestimmt. Die beispielhaften Formkörper hatten eine Dichte von durchschnittlich ca. 1,1g/cm³. Die Messung erfolgte auf einer Universalprüfmaschine der Fa. Hegewald&Peschke unter Verwendung einer 20kN Kraftmesszelle bei einer Vorschubgeschwindigkeit von 1 mm/min. Die stoffliche Umsetzung in Abhängigkeit von der Aushärtedauer bei 50°C und gesättigter Luftfeuchtigkeit wurde mittels XRD-Messungen (Röntgendiffraktogrammen) untersucht.

**Tabelle 1: Druckfestigkeitsmittelwerte (DF-MW) von je 7 Probekörpern, die bei 50°C und gesättigter Luftfeuchte ausgehärtet wurden.**

| **Zeit** | **DF-MW [MPa]** |
|---|---|
| 6 h | 1,72 |
| 16 h | 5,25 |
| 24 h | 5,85 |
| 38 h | 6,40 |
| 48 h | 11,44 |
| 4 d | 10,38 |
| 7 d | 10,64 |
| 14 d | 11,50 |

Die gemeinsame Betrachtung des Druckfestigkeitsverlaufs und der stofflichen Umsetzung der mineralischen Phasen im Zuge der Abbindereaktion zeigen, dass die Zunahme der mechanischen Festigkeit deutlich schneller erfolgt, als nach der stofflichen Umsetzung der mineralischen Ausgangskomponenten zu Hydroxylapatit, dem Reaktionsprodukt der Abbindereaktion, zu erwarten wäre. Insbesondere zeigen die Druckfestigkeitswerte nach 48h bereits den Maximalwert der Druckfestigkeit, während die stoffliche Umsetzung der reaktiven Ausgangskomponenten noch bei weitem nicht abgeschlossen ist und anhand des Röntgendiffraktogramms bei < 50% liegt.

Der Grad der Umsetzung wird in dieser Gegenüberstellung anhand des röntgendiffraktometrisch bestimmten Gehalts an α-TCP bzw. dem Verhältnis von α-TCP zu Hydroxylapatit zu den jeweiligen Messzeitpunkten bestimmt (Fig. 1a und 1b). Der Anfangsgehalt aller reaktiven mineralischen Komponenten betrug 96%, wobei der Anfangsgehalt an α-TCP in der reaktiven mineralischen Pulvermischung 60% betrug. Nach 48h wurde der Gehalt an α-TCP zu ca. 45% bestimmt, während sich der Restgesamtgehalt aller reaktiven mineralischen Pulverbestandteile auf ca. 50% beläuft. Nach 14 Tagen ist die stoffliche Umsetzung zu Hydroxylapatit unter den gewählten Bedingungen fast vollständig abgeschlossen, es sind lediglich noch Spuren von α-TCP röntgendiffraktometrisch nachzuweisen (Fig. 1b). Ein weiterer Festigkeitsanstieg ist mit der fortschreitenden Umsetzung allerdings nicht verbunden.

Die Betrachtung des Druckfestigkeitsverlaufs ermöglicht die gezielte Auswahl von Produkteigenschaften in Bezug auf die vorgesehene Anwendung. Bei einer erwünschten möglichst einfachen Bearbeitbarkeit und der angestrebten weitestgehenden Umsetzung unter biologischen Bedingungen würde die Aushärtung zu einem frühen Zeitpunkt durch Wasserentzug gestoppt. Im Falle der beispielhaften Formkörper könnte dies bereits nach ca. 6h erfolgen, da zu diesem Zeitpunkt bereits eine ausreichende Formstabilität des gedruckten Formkörpers erreicht ist, wobei eine Festigkeit von ca. 2MPa angestrebt wird. Bevorzugt ist ein Abbruch der Abbindereaktion nach ca. 16h, wenn die Festigkeit ca. 50% der maximalen Festigkeit beträgt. Besonders bevorzugt ist ein Abbruch der Abbindereaktion im Falle der beispielhaften Formkörper nach ca. 48h, wenn die Festigkeit 100% oder annähernd 100% der maximalen Festigkeit erreicht hat. Dabei wird der Zeitpunkt des Abbruchs der Abbindereaktion so gewählt, dass zu diesem Zeitpunkt ein möglichst geringer Wert für die stoffliche Umsetzung vorliegt. Experimentelle Daten haben ergeben, dass dieser Zusammenhang bei dem beispielhaften Formkörper bei ca. 48h erreicht wird. Auf diese Weise kann ein optimaler Kompromiss zwischen Festigkeit und biologischer Aktivität erreicht werden.

### 1.6. Umsetzung der Formkörper mit verbleibender hydraulischer Aktivität nach Inkubation in unterschiedlichen Medien

Formkörper der Dimension 10x10x5 mm mit der stofflichen Zusammensetzung nach Ausführungsbeispiel 1.1 wurden im 3D-Druckverfahren nach Ausführungsbeispiel 1.2 und einer Aushärtung über 24h nach Ausführungsbeispiel 1.3 und einem Abbruch der Aushärtung durch Wasserentzug nach Ausführungsbeispiel 1.4 so hergestellt, dass die durchschnittliche Dichte bei 1,5 g/cm³ lag. Anschließend wurden die Formkörper in Folienbeutel luftdicht verpackt und bis zur weiteren Untersuchung bei Raumtemperatur gelagert. Die so hergestellten Formkörper hatten eine Druckfestigkeit von 7,8 MPa. Jeweils 3 der Formkörper (pro Medium und Untersuchungszeitpunkt) wurden in unterschiedliche Medien (NaCl, DMEM (Zellkulturmedium) und SBF (simulated body fluid)) eingelegt, um den Einfluss der Medienzusammensetzung auf die weitere Aushärtung der Formkörper zu untersuchen, die Inkubation erfolgte bei 37°C. Fig. 2 zeigt den Festigkeitsverlauf nach unterschiedlichen Inkubationszeiten. Die Festigkeit der Formkörper nimmt schon nach kurzer Inkubationszeit in allen Medien stark zu. Der Anstieg geht über den gesamten Untersuchungszeitraum von 168 Tagen weiter und erreicht mit ca. 30 - 35 MPa unerwartet hohe Werte. Zwischen den unterschiedlichen Inkubationsmedien, die sich in der Zusammensetzung deutlich unterscheiden, gibt es in Bezug auf den Festigkeitsverlauf keine signifikanten Unterschiede. Die Ergebnisse zeigen, dass die durch Wasserentzug unterbrochene Abbindereaktion nach erneuter Einlagerung in wässrige Medien fortgesetzt wird und dass die weitere Reaktion weitgehend unabhängig von der Zusammensetzung des Mediums ist (sofern sich die Zusammensetzung des Mediums im biologisch relevanten Rahmen befindet).

### Ausführungsbeispiel 2 - Implantationsstudie im Schaf

Gedruckte Formkörper nach Beispiel 1 nach 4 Tagen Aushärtung wurden mittels Fräsen zu Halbkugeln mit einem Radius von 5mm geformt, anschließend in Wasser und Aceton gewaschen und getrocknet und danach einzeln in Kunststoffröhrchen verpackt und mit 25 kGy durch Gammabestrahlung sterilisiert. Nach der Technik nach Busenlechner (Biomaterials 29 (2008) 3195-3200) wurden die Halbkugeln aus den gedruckten Formkörpern formschlüssig in Titanhalbsphären mit einer Innenweite von 10mm eingebracht und nach Setzen von jeweils 11 kleinen Bohrungen von 1mm Ø und 2mm Tiefe auf die Calvarien von ausgewachsenen Schafen aufgesetzt. Die Kontrollgruppe enthielt Halbsphären, die mit porösem ß-TCP (Knochenersatzmaterial nach dem Stand der Technik) gefüllt waren. Nach 8 und 16 Wochen wurden die Implantate entnommen und histologisch ausgewertet. Die Gruppe mit den gedruckten Formkörpern zeigte eine signifikant stärkere Bildung von neuem Knochen als die Gruppe mit ß-TCP. Während praktisch alle Poren der gedruckten Formkörper mit neuem Knochen gefüllt waren und alle Oberflächen des erfindungsgemäßen Materials mit neuem Knochen überzogen waren, war dies im Falle des ß-TCP nur sporadisch der Fall. Diese in vivo Ergebnisse zeigen die außergewöhnlich hohe Bioaktivität des erfindungsgemäßen Materials im Vergleich zu einem in der Orthopädie und Traumatologie standardmäßig verwendeten Knochenersatzformkörper. Die Ergebnisse der Studie bestätigen, dass die Bildung von nanokristallinem Hydroxylapatit unter biologischen Bedingungen zu einem Material mit besonders hoher Bioaktivität führt. Diese hohe Bioaktivität schlägt sich in einer signifikant erhöhten Rate an Knochenneubildung in einem klinisch relevanten Modell zur Knochenheilung nieder. Als Vergleichsimplantat diente ein ß-TCP, dass im molaren Calcium/Phosphat-Verhältnis (ca. 1,5) und in der Gesamtporosität mit dem erfindungsgemäßen Implantatmaterial vergleichbar ist. Die signifikant erhöhte Bildung neuen Knochens und die ebenfalls signifikant schnellere und vollständigere knöcherne Integration können daher ursächlich auf die höhere Bioaktivität durch die erfindungsgemäße Zusammensetzung und Herstellungsweise der beschriebenen Formkörper mit verbleibender hydraulischer Aktivität zurückgeführt werden.

### Ausführungsbeispiel 3 - Formkörper mit verbleibender hydraulischer Aktivität auf der Basis von Magnesium-Calcium-Phosphat-Zement (MgCPC)

Analog zu Ausführungsbeispiel 1 wurden Zementpasten auf der Basis von MgCPC hergestellt, indem bei gleicher organischer Phase die mineralische Phase aus MgCPC-Pulver mit der Zusammensetzung (Ca_{0,5}Mg_{2,5}(PO₄)₂) in einem Verhältnis von 84 Gew.-% Pulver zu 16 Gew.-% Trägerflüssigkeit hergestellt wurde. Die Formgebung erfolgte ebenfalls analog zu Beispiel 1 mittels 3D-Drucker zu Formkörpern der Dimension 10x10x5 mm mit einer mittleren Dichte von 1,4 g/ml. Die initiale Aushärtung der Formkörper erfolgte in einer gesättigten Wasserdampfatmosphäre bei 37°C über 24 h. Anschließend wurden die Formkörper in unterschiedlichen Medien inkubiert, um den Einfluss der Medienzusammensetzung zu untersuchen. In Fig. 3 ist die Druckfestigkeit der Formkörper in Abhängigkeit vom pH-Wert der Inkubationslösung dargestellt. Das Ergebnis zeigt, dass MgCPC-Formkörper mit verbleibender hydraulischer Aktivität mit relativ hoher initialer Festigkeit hergestellt werden können, die anschließend (nach Abbruch der Aushärtung durch Wasserentzug) durch Inkubation über 24 h in definierter Pufferlösung weiter verfestigt werden können (die Referenz hatte eine Druckfestigkeit von 17 MPa).

### Ausführungsbeispiel 4 - Abbinden von Formkörpern über mehrere Stufen

CPC-Formkörper der Dimension 6x6x12 mm werden durch Einstreichen von Paste-CPC in eine teilbare Metallform präpariert, indem sie über 24 h bei 37°C in 0,9%ige NaCI-Lösung eingelegt werden. Nach der Entformung werden die Formkörper in destilliertem Wasser gewaschen und getrocknet. Die Druckfestigkeit beträgt ca. 12 MPa. Auf einer Drehmaschine werden aus diesen geringgradig abgebundenen Formkörpern Zylinder der Dimension 5 mm Ø und 12 mm Höhe hergestellt. Anschließend werden die Zylinder über 2 Tage bei 37°C in 1%ige CaCL2-Lösung inkubiert, mit Aceton gewaschen und getrocknet. Die Druckfestigkeit beträgt 32 MPa. Die so erhaltenen Formkörper werden anschließend in simulierter Körperflüssigkeit bei 37°C über 7 Tage eingelegt, anschließend in Aceton entwässert und getrocknet. Die Druckfestigkeit beträgt 56 MPa. Der Umsetzungsgrad beträgt nach XRD-Analyse ca. 85%.

Die Formkörper können - abhängig vom Verwendungszweck - nach jedem der beschriebenen Schritte als Produkt verwendet werden.

## Patentansprüche

1. Formstabile Knochenersatzformkörper aus mineralischem Knochenzement mit verbleibender hydraulischer Aktivität, enthaltend
- mindestens einen Anteil an abgebundenem mineralischem Knochenzement,
- mindestens einen Anteil an nicht umgesetztem oder nicht abgebundenem reaktivem mineralischem Knochenzement,
erhältlich nach einem Verfahren umfassend die folgenden Schritte:
a. Anmischen eines reaktiven mineralischen Knochenzementes mit einer schwer wasserlöslichen Trägerflüssigkeit zu einer formbaren Knochenzementmasse,
b. Formgebung der Knochenzementmasse zu einem Knochenersatzformkörper, wobei die Formgebung der Knochenzementmasse durch ein (3D-) Druckverfahren oder über einen Granulationsprozess erfolgt,
c. Kontaktieren des Knochenersatzformkörpers mit einer wässrigen Lösung oder einer Wasser-(dampf-)gesättigten Umgebung, so dass ein Abbindeprozess initiiert wird und der Knochenersatzformkörper mindestens 5% des Maximalwerts der Festigkeit eines vollständig abgebundenen Knochenersatzmaterials aus den gleichen Komponenten und mit den gleichen Strukturmerkmalen, insbesondere gleicher Porosität, erreicht,
d. Abbruch des Abbindeprozesses durch den substantiellen Entzug von Wasser, so dass der formstabile Knochenersatzformkörper, der unter biologischen Bedingungen - 37°C und Wassersättigung - umgesetzt werden kann, einen Anteil an abgebundenem mineralischem Knochenzement von 5 bis 90-Ma % enthält und dass der formstabile Knochenersatzformkörper substantiell wasserfrei ist,
wobei die formstabilen Knochenersatzformkörper Druckfestigkeiten im Bereich von 2 bis 200 MPa aufweisen.

2. Formstabile Knochenersatzformkörper nach Anspruch 1, **dadurch gekennzeichnet, dass** der mindestens eine Anteil an nicht umgesetztem oder nicht abgebundenem reaktivem mineralischem Knochenzement mindestens eine nicht umgesetzte oder nicht abgebundene reaktive mineralische Knochenzementkomponente mit einem Anteil von mindestens 10 Ma.-% enthält.

3. Formstabile Knochenersatzformkörper nach Anspruch 1 oder 2, **dadurch gekennzeichnet, dass** die formstabilen Knochenersatzformkörper eine Gesamtporosität zwischen 20 und 90% aufweisen.

4. Formstabile Knochenersatzformkörper nach einem der vorangegangenen Ansprüche in Form von Granulaten, wobei die einzelnen Granula eine Größe von >100 µm und <10 mm aufweisen.

5. Verfahren zur Herstellung formstabiler Knochenersatzformkörper mit verbleibender hydraulischer Aktivität, umfassend die folgenden Schritte:
a. Anmischen eines reaktiven mineralischen Knochenzementes mit einer schwer wasserlöslichen Trägerflüssigkeit zu einer formbaren Knochenzementmasse,
b. Formgebung der Knochenzementmasse zu einem Knochenersatzformkörper, wobei die Formgebung der Knochenzementmasse durch ein (3D-) Druckverfahren oder über einen Granulationsprozess erfolgt,
c. Kontaktieren des Knochenersatzformkörpers mit einer wässrigen Lösung oder einer Wasser-(dampf-)gesättigten Umgebung, so dass ein Abbindeprozess initiiert wird und der Knochenersatzformkörper mindestens 5% des Maximalwerts der Festigkeit eines vollständig abgebundenen Knochenersatzmaterials aus den gleichen Komponenten und mit den gleichen Strukturmerkmalen, insbesondere gleicher Porosität, erreicht,
d. Abbruch des Abbindeprozesses durch den substantiellen Entzug von Wasser, so dass der formstabile Knochenersatzformkörper, der unter biologischen Bedingungen - 37°C und Wassersättigung - umgesetzt werden kann, einen Anteil an abgebundenem mineralischem Knochenzement von 5 bis 90-Ma % enthält und dass der formstabile Knochenersatzformkörper substantiell wasserfrei ist.

6. Verfahren nach Anspruch 5, **dadurch gekennzeichnet, dass** der Knochenersatzformkörper durch die Entfernung von enthaltenen Hilfsstoffen, Restwasser und/oder wasserlöslichen Lösungsmitteln, die zum substantiellen Entzug des Wassers verwendet wurden, getrocknet wird.

7. Verfahren nach Anspruch 5 oder 6, **dadurch gekennzeichnet, dass** als mindestens eine reaktive mineralische Knochenzementkomponente zum Anmischen des reaktiven mineralischen Knochenzementes eine Substanz aus der Gruppe der Silikate, Phosphate, Sulfate, Carbonate, Oxide und/oder Hydroxide in Verbindung mit Calciumionen, Magnesiumionen und/oder Strontiumionen verwendet wird, die bei dem Kontaktieren mit einer wässrigen Lösung oder nach Einbringung in eine wässrige Lösung in einem hydraulischen Abbindeprozess zu einem schwerlöslichen Festkörper abbindet.

8. Verfahren nach einem der Ansprüche 5 bis 7, **dadurch gekennzeichnet, dass** beim Anmischen des reaktiven mineralischen Knochenzements zu einer formbaren Knochenzementmasse Zusatzstoffe verwendet werden.

9. Verfahren nach einem der Ansprüche 5 bis 8, **dadurch gekennzeichnet, dass** die wässrige Lösung mindestens einen Zusatzstoff ausgewählt aus einer Pufferlösung, einem organischen und/oder anorganischen Salz, einer Zellpräparation, einem biologischen, rekombinanten oder pharmakologischen Wirkstoff, einer Nukleinsäure (RNA oder DNA), Mischungen von Nukleinsäuren, einer Aminosäure, einer modifizierten Aminosäure, einem Vitamin und Mischungen aus diesen, enthält.

10. Verfahren nach einem der Ansprüche 5 bis 9, **dadurch gekennzeichnet, dass** die Initiation des Abbindeprozesses in einer Wasserdampfatmosphäre bei > 90% relativer Luftfeuchtigkeit und einer Temperatur zwischen 0 und 100°C erfolgt.

11. Verfahren nach einem der Ansprüche 5 bis 10, **dadurch gekennzeichnet, dass** der Abbruch des Abbindeprozesses durch den substanziellen Entzug von Wasser bei einer Temperatur zwischen 0 und 100°C erfolgt.

12. Verfahren nach einem der Ansprüche 5 bis 11, **dadurch gekennzeichnet, dass** die Abbindereaktion der Formkörper in mehreren Schritten und/oder mit verschiedenen wässrigen Lösungen und/oder Wasserdampfatmosphäre durchgeführt wird.

13. Verwendung eines formstabilen Knochenersatzformkörpers nach einem der vorangegangenen Ansprüche zur Herstellung eines alloplastischen Implantats.

14. Verwendung eines formstabilen Formkörpers nach einem der Ansprüche 1-12 Trägermaterial in der Zellkultur, der Gewebekultur und/oder des Tissue Engineering.

## Claims

1. Dimensionally stable bone replacement moulded bodies made of mineral bone cement and having residual hydraulic activity, containing
- at least one proportion of set mineral bone cement,
- at least one proportion of unreacted or unset reactive mineral bone cement,
obtainable according to a method comprising the following steps:
a. mixing a reactive mineral bone cement with a low water-soluble carrier liquid to form a mouldable bone cement compound;
b. shaping the bone cement compound to form a bone replacement moulded body; wherein the bone cement compound is shaped using a 3D printing method or by means of a granulation process;
c. contacting the bone replacement moulded body with an aqueous solution or a water (vapour)-saturated environment such that a setting process is initiated and the bone replacement moulded body reaches at least 5% of the maximum value of the strength of a completely set bone replacement material made of the same components and having the same structural features, in particular the same porosity;
d. discontinuing the setting process by the substantial removal of water, such that the dimensionally stable bone replacement moulded body, which can be reacted under biological conditions (37°C and water saturation), contains a proportion of set mineral bone cement of from 5 to 90 m.% and that the dimensionally stable bone replacement moulded body is substantially free of water,
wherein the dimensionally stable bone replacement moulded bodies have compressive strengths in the range of from 2 to 200 MPa.

2. Dimensionally stable bone replacement moulded bodies according to claim 1, **characterised in that** the at least one proportion of unreacted or unset reactive mineral bone cement contains at least one unreacted or unset reactive mineral bone cement component having a proportion of at least 10 m.%.

3. Dimensionally stable bone replacement moulded bodies according to either claim 1 or claim 2, **characterised in that** the dimensionally stable bone replacement moulded bodies have a total porosity of between 20 and 90%.

4. Dimensionally stable bone replacement moulded bodies according to any of the preceding claims in the form of granules, wherein the individual granules have a size of > 100 µm and < 10 mm.

5. Method for producing dimensionally stable bone replacement moulded bodies having residual hydraulic activity, comprising the following steps:
a. mixing a reactive mineral bone cement with a low water-soluble carrier liquid to form a mouldable bone cement compound;
b. shaping the bone cement compound to form a bone replacement moulded body, wherein the bone cement compound is shaped using a 3D printing method or by means of a granulation process;
c. contacting the bone replacement moulded body with an aqueous solution or a water (vapour)-saturated environment such that a setting process is initiated and the bone replacement moulded body reaches at least 5% of the maximum value of the strength of a completely set bone replacement material made of the same components and having the same structural features, in particular the same porosity;
d. discontinuing the setting process by the substantial removal of water, such that the dimensionally stable bone replacement moulded body, which can be reacted under biological conditions (37°C and water saturation), contains a proportion of set mineral bone cement of from 5 to 90 m.% and that the dimensionally stable bone replacement moulded body is substantially free of water.

6. Method according to claim 5, **characterised in that** the bone replacement moulded body is dried by removing contained additives, residual water and/or water-soluble solvents which have been used for the substantial removal of water.

7. Method according to either claim 5 or claim 6, **characterised in that** a substance from the group of silicates, phosphates, sulfates, carbonates, oxides and/or hydroxides in combination with calcium ions, magnesium ions and/or strontium ions is used as the at least one reactive mineral bone cement component for blending the reactive mineral bone cement, which substance sets on contact with an aqueous solution or after introduction into an aqueous solution in a hydraulic setting process to form a poorly soluble solid.

8. Method according to any of claims 5 to 7, **characterised in that** additives are used when blending the reactive mineral bone cement to form a mouldable bone cement compound.

9. Method according to any of claims 5 to 8, **characterised in that** the aqueous solution contains at least one additive selected from a buffer solution, an organic and/or inorganic salt, a cell preparation, a biological, recombinant or pharmacological active ingredient, a nucleic acid (RNA or DNA), mixtures of nucleic acids, an amino acid, a modified amino acid, a vitamin and mixtures thereof.

10. Method according to any of claims 5 to 9, **characterised in that** the setting process is initiated in a water vapour atmosphere at > 90% relative air humidity and at a temperature of between 0 and 100°C.

11. Method according to any of claims 5 to 10, **characterised in that** the setting process is discontinued by the substantial removal of water at a temperature of between 0 and 100°C.

12. Method according to any of claims 5 to 11, **characterised in that** the setting reaction of the moulded bodies is carried out in a plurality of steps and/or with different aqueous solutions and/or water vapour atmospheres.

13. Use of a dimensionally stable bone replacement moulded body according to any of the preceding claims for producing an alloplastic implant.

14. Use of a dimensionally stable moulded body according to any of claims 1 to 12 as a carrier material in cell culture, tissue culture and/or tissue engineering.

## Revendications

1. Eléments moulés de substitution osseuse de forme stable en ciment osseux minéral ayant une activité hydraulique résiduelle, contenant
- au moins une proportion de ciment osseux minéral ayant pris,
- au moins une proportion de ciment osseux minéral réactif n'ayant pas réagi ou n'ayant pas pris,
pouvant être obtenu selon un procédé comprenant les étapes suivantes :
a. mélange d'un ciment osseux minéral réactif avec un liquide porteur difficilement soluble dans l'eau pour donner une masse de ciment osseux malléable,
b. façonnage de la masse de ciment osseux pour donner un élément moulé de substitution osseuse, dans lequel le façonnage de la masse de ciment osseux est effectué par un procédé d'impression (3D) ou par le biais d'un processus de granulation,
c. mise en contact de l'élément moulé de substitution osseuse avec une solution aqueuse ou un environnement saturé en (vapeur d') eau de telle sorte qu'un processus de prise est lancé, et l'élément moulé de substitution osseuse atteint au moins 5 % de la valeur maximale de la solidité d'un matériau de substitution osseuse ayant entièrement pris composé des mêmes composants et ayant les mêmes caractéristiques structurelles, en particulier la même porosité,
d. arrêt du processus de prise par le retrait substantiel d'eau, de telle sorte que l'élément moulé de substitution osseuse de forme stable qui peut réagir en présence de conditions biologiques - 37 °C et saturation en eau - contient une proportion de ciment osseux minéral ayant pris de 5 à 90 % en masse, et de telle sorte que l'élément moulé de substitution osseuse de forme stable est substantiellement dépourvu d'eau,
dans lequel les éléments moulés de substitution osseuse de forme stable présentent des résistances à la pression dans la plage de 2 à 200 MPa.

2. Eléments moulés de substitution osseuse de forme stable selon la revendication 1, **caractérisés en ce que** l'au moins une proportion de ciment osseux minéral réactif n'ayant pas réagi ou n'ayant pas pris contient au moins une composante de ciment osseux minérale réactive n'ayant pas réagi ou n'ayant pas pris ayant une proportion d'au moins 10 % en masse.

3. Eléments moulés de substitution osseuse de forme stable selon la revendication 1 ou 2, **caractérisés en ce que** les éléments moulés de substitution osseuse de forme stable présentent une porosité globale entre 20 et 90 %.

4. Eléments moulés de substitution osseuse de forme stable selon l'une des revendications précédentes sous forme de granulats, les différents granulés présentant une dimension de > 100 µm et < 10 mm.

5. Procédé de fabrication d'éléments moulés de substitution osseuse de forme stable ayant une activité hydraulique résiduelle, comprenant les étapes suivantes :
a. mélange d'un ciment osseux minéral réactif avec un liquide porteur difficilement soluble dans l'eau pour donner une masse de ciment osseux malléable,
b. façonnage de la masse de ciment osseux pour donner un élément moulé de substitution osseuse, dans lequel le façonnage de la masse de ciment osseux est effectué par un procédé d'impression (3D) ou par le biais d'un processus de granulation,
c. mise en contact de l'élément moulé de substitution osseuse avec une solution aqueuse ou un environnement saturé en (vapeur d') eau de telle sorte qu'un processus de prise est lancé, et l'élément moulé de substitution osseuse atteint au moins 5 % de la valeur maximale de la solidité d'un matériau de substitution osseuse ayant entièrement pris composé des mêmes composants et ayant les mêmes caractéristiques structurelles, en particulier la même porosité,
d. arrêt du processus de prise par le retrait substantiel d'eau, de telle sorte que l'élément moulé de substitution osseuse de forme stable qui peut réagir en présence de conditions biologiques - 37 °C et saturation en eau - contient une proportion de ciment osseux minéral ayant pris de 5 à 90 % en masse, et de telle sorte que l'élément moulé de substitution osseuse de forme stable est substantiellement dépourvu d'eau.

6. Procédé selon la revendication 5, **caractérisé en ce que** l'élément moulé de substitution osseuse est séché par l'enlèvement d'adjuvants contenus, d'eau résiduelle et/ou de solvants solubles dans l'eau qui ont été utilisés pour le retrait substantiel de l'eau.

7. Procédé selon la revendication 5 ou 6, **caractérisé en ce que**, en tant qu'au moins une composante de ciment osseux minérale réactive pour le mélange du ciment osseux minéral réactif, il est utilisé une substance issue du groupe des silicates, phosphates, sulfates, carbonates, oxydes et/ou hydroxydes en liaison avec des ions calcium, des ions magnésium et/ou des ions strontium qui, lors de la mise en contact avec une solution aqueuse ou après la mise en place dans une solution aqueuse, prend dans un processus de prise hydraulique pour donner un solide difficilement soluble.

8. Procédé selon l'une des revendications 5 à 7, **caractérisé en ce que** des additifs sont utilisés lors du mélange du ciment osseux minéral réactif pour donner une masse de ciment osseux malléable.

9. Procédé selon l'une des revendications 5 à 8, **caractérisé en ce que** la solution aqueuse contient au moins un additif sélectionné parmi une solution tampon, un sel organique et/ou inorganique, une préparation cellulaire, une substance active biologique, recombinante ou pharmacologique, un acide nucléique (ARN ou ADN), des mélanges d'acides nucléiques, un acide aminé, un acide aminé modifié, une vitamine et des mélanges de ceux-ci.

10. Procédé selon l'une des revendications 5 à 9, **caractérisé en ce que** le lancement du processus de prise s'effectue dans une atmosphère de vapeur d'eau en présence de > 90 % d'humidité relative de l'air et d'une température entre 0 et 100 °C.

11. Procédé selon l'une des revendications 5 à 10, **caractérisé en ce que** l'arrêt du processus de prise s'effectue par le retrait substantiel d'eau en présence d'une température entre 0 et 100 °C.

12. Procédé selon l'une des revendications 5 à 11, **caractérisé en ce que** la réaction de prise des éléments moulés est réalisée en plusieurs étapes et/ou avec différentes solutions aqueuses et/ou avec une atmosphère de vapeur d'eau.

13. Utilisation d'un élément moulé de substitution osseuse de forme stable selon l'une des revendications précédentes pour la fabrication d'un implant alloplastique.

14. Utilisation d'un élément moulé de forme stable selon l'une des revendications 1-12 en tant que matériau support dans la culture cellulaire, la culture tissulaire et/ou de l'ingénierie tissulaire.
